(19) Europäisches Patentamt
European Patent Office
Office européen des brevets

(11) **EP 1 684 710 B1**

(12) **EUROPÄISCHE PATENTSCHRIFT**

(45) Veröffentlichungstag und Bekanntmachung des Hinweises auf die Patenterteilung:
**17.12.2008 Patentblatt 2008/51**

(21) Anmeldenummer: **04790872.8**

(22) Anmeldetag: **21.10.2004**

(51) Int Cl.:
*A61K 8/81* (2006.01)    *A61K 8/85* (2006.01)
*A61Q 1/00* (2006.01)

(86) Internationale Anmeldenummer:
**PCT/EP2004/012089**

(87) Internationale Veröffentlichungsnummer:
**WO 2005/039512 (06.05.2005 Gazette 2005/18)**

(54) **KLIMABESTÄNDIGER KOSMETISCHER KOMPLEX**

CLIMATE-RESISTANT COSMETIC COMPLEX

COMPLEXE COSMETIQUE RESISTANT AU CLIMAT

(84) Benannte Vertragsstaaten:
**AT BE BG CH CY CZ DE DK EE ES FI FR GB GR HU IE IT LI LU MC NL PL PT RO SE SI SK TR**

(30) Priorität: **23.10.2003 DE 10350322**

(43) Veröffentlichungstag der Anmeldung:
**02.08.2006 Patentblatt 2006/31**

(73) Patentinhaber: **Coty B.V.**
**2031 CC Haarlem (NL)**

(72) Erfinder:
• **HWANG, Donna Hui-Ing**
**Leonia, NJ 07605 (US)**
• **CERNASOV, Domnica**
**Ringwood, NJ 07456 (US)**
• **MACCHIO, Ralph**
**Sparta, NJ 07971 (US)**

(74) Vertreter: **Walter, Wolf-Jürgen et al**
**Anwaltskanzlei**
**Gulde Hengelhaupt Ziebig & Schneider**
**Wallstrasse 58/59**
**10179 Berlin (DE)**

(56) Entgegenhaltungen:
**EP-A- 1 013 256**          **WO-A-20/04066918**
**US-A1- 2003 165 451**

Bemerkungen:
Die Akte enthält technische Angaben, die nach dem Eingang der Anmeldung eingereicht wurden und die nicht in dieser Patentschrift enthalten sind.

EP 1 684 710 B1

**Beschreibung**

Die Erfindung betrifft einen klimabeständigen kosmetischen Komplex mit langanhaltender Feuchthaltewirkung und Wasserbeständigkeit.

Aus dem Stand der Technik sind bereits wasserbeständige Produkte bekannt, die auf dem Gebiet der dekorativen Kosmetik eingesetzt werden. In der EP-A-1013256 ist eine Wimperntusche beschrieben, bei der eine wässrige Phase in einer flüssigen Fettphase dispergiert ist, wobei die wässrige Phase auch ein filmbildendes Polymersystem als feste Teilchen enthält sowie lamellare Füllstoffe. WO-A-2004/066918 beschreibt langhaltende, nichtwässrige kosmetische Zusammensetzungen, die mindestens ein Emulgator enthalten. Bei WO-A-2004/066918 handelt es sich um ein Dokument gemäß Artikel 54(3) EPÜ.

Die US-A1-2003/0165451 beschreibt eine W/O-Emulsion zur Verwendung als schützende Hautcrème, bei der die üblicherweise eingesetzten Wachse durch semikristalline Polymere ersetzt werden und somit ein besonders angenehmes Hautgefühl erzeugt wird. Die EP 925778 B1 beschreibt eine wässrige Emulsion von festen Silikonverbindungen in Kombination mit filmbildenden Polymeren, die ein wasserbeständiges Pflegeprodukt für Haut und Lippen darstellen. In der EP 1064930 B1 wird u.a. ein Make-up beschrieben, bestehend aus einer wässrigen Phase und darin dispergierten Fasern und einem vernetzten, festen, elastomeren Polyorgano-siloxan. Diese wasserbeständigen Zusammensetzungen haben eine geringe Hautaffinität und können leicht von der Haut abgewaschen werden, wodurch keine Langzeit-Wasserbeständigkeit erreicht werden kann.

Aufgabe der vorliegenden Erfindung ist die Bereitstellung eines klimabeständigen Kosmetikkomplexes mit langanhaltender Feuchthaltewirkung und zugleich ausgezeichneter Langzeit-Wasserbeständigkeit und Übertragungsbeständigkeit.

Erfindungsgemäß besteht der klimabeständige kosmetische Komplex aus

0,1 - 90 Gew.-% einer gelierten Ölzusammensetzung, bestehend aus einer Ölkomponente und einer Polymerkomponente, wobei die Poly merkomponente aus der Gruppe ausgewählt ist, bestehend aus Tri-Block-Copolymeren, Sternpolymeren, Radialpolymeren, Multi-Block-Polymeren von Polystyrol, Polyethylen, Polyvinylchlorid, Polyisopren, Polybutadien, Ethylen/Butadien-Copolymeren, Ethylen/- Propylen-Copolymeren, Ethylen/Butylen-Copolymeren, Ethylen-Propylen/Dien-Copolymeren, Styrol-Ethylen/Propylen-Copolymeren, Styrol-Ethylen/Butadien-Copolymeren, Styrol-Isopren-Copolymeren, Styrol-Butadien-Copolymeren, Styrol-Ethylen/Propylen-Styren-Copolymeren, Styrol-Ethylen/Butadien-Styren-Copolymeren, Styrol-Isopren-Styren-Copolymeren, Styrol-Butadien-Styren-Copolymeren und Gemischen davon;

0,1 - 80 Gew.-% eines topischen wasserabweisenden vernetzten Polyesters mit einem Molekulargewicht von 600 - 8000, bestehend aus mehrwertigen Alkoholen und Dicarbonsäuren;

0,01 - 20 Gew.-% eines wasserabsorbierenden Puders mit einer Teilchengröße von 1 bis 100 $\mu$m, wobei der Puder aus der Gruppe ausgewählt ist, bestehend aus Pudern auf Basis natürlicher pflanzlicher zellulosereicher Bestandteile, Maltodextrin, Stärke, Stärke/Polyacrylat-Copolymere, aus Acrylmonomeren hergestellte synthetische Polymere und Gemische davon;

0,01 - 20 Gew.-% eines Verdickungsmittels; und

0,1 - 50 Gew.-% organische Lösungsmittel, Trägerstoffe oder Gemische davon, jeweils bezogen auf das Gesamtgewicht des Komplexes und der Komplex enthält keinen Emulgator.

Unter klimabeständigem kosmetischem Komplex wird ein Hautschutz-komplex gegen witterungsbedingte negative Umwelteinflüsse verstanden. Der Komplex bildet einen an der Haut haftenden wasserbeständigen Film und verhindert das Eindringen von mit Regen oder Schnee einhergehenden unerwünschten Fremdkörpern wie Stäuben und Umweltschmutz in die Haut. Der Komplex zeigt außerdem eine verlängerte wasserhaltende Fähigkeit, die eine Hautdehydratisierung durch Wind oder Sonne verhindert.

Für die gelierte Ölzusammensetzung in dem erfindungsgemmäßen Komplex ist ein bevorzugter Bereich 20 bis 70 Gew.-%.

Der Polymeranteil in der Ölzusammensetzung liegt vorteilhaft im Bereich von 1:5 bis 1:50 Polymerkomponente: Ölkomponente.

Die Ölkomponente ist z.B. ausgewählt unter Kohlenwasserstoffen, Fettalkoholen, natürlichen und synthetischen Ölen, Estern, Ethern und Gemischen davon.

Die Polymerkomponente ist, wie bereits ausgeführt, ausgewählt unter Tri-Block-Copolymeren, Sternpolymeren, Radialpolymeren, Multi-Block-Polymeren und Kombinationen davon. Diese synthetischen Polymere oder Copolymere sind z.B. Polystyrol, Polyethylen, Polyvinylchlorid, Polyisopren, Polybutadien, Ethylen/Butadien-Copolymere, Ethylen/Propylen-Copolymere, Ethylen/Butylen-Copolymere, Ethylen-Propylen/Dien-Copolymere, Styrol-Ethylen/Propylen-Copolymere, Styrol-Ethylen/Butadien-Copolymere, Styrol-Isopren-Copolymere, Styrol-Butadien-Copolymere, Styrol-Ethylen/Propylen-Styren-Copolymere, Styrol-Ethylen/Butadien-Styren-Copolymere, Styrol-Isopren-Styren-Copolymere, Styrol-Butadien-Styren-Copolymere und Gemische davon. Besonders bevorzugt sind ein oder mehrere Diblockcopolymere, ein oder mehrere Triblockcopolymere und Gemische davon.

Besonders bevorzugt für die Polymerkomponente sind 20 bis 70 Gew-%.

Für die topische wasserabweisende Substanz ist Voraussetzung, dass sie eine Affinität zur Haut haben muss, in Wasser nicht löslich ist und eine lange Beständigkeit in Wasser hat. Außerdem ist sie in der Lage, puderförmige Substanzen zurückzuhalten und damit die oberen Schichten der Epidermis zu schützen.

Die topische wasserabweisende Substanz hat ein Molekulargewicht im Bereich von 600 - 8000 und ist ein vernetzter Polyester aus mehrwertigen Alkoholen und Dicarbonsäuren.

Mehrwertige Alkohole sind z.B. Trimethylpentandiol, Glycerin oder Diethylenglycol.

Zu den Säuren gehören z.B. Adipinsäure und Fettsäuren. Eine bevorzugte wasserabweisende Substanz ist z. B. Trimethylpentandiol/Adipinsäure/Glycerin-Copolymer.

Die gelierte Ölzusammensetzung und die topische wasserabweisende Substanz ergänzen sich in ihren Eigenschaften dahingehend, dass die starke Hautaffinität, Übertragungsbeständigkeit und Klebrigkeit der topischen wasserabweisenden Substanz und die wasserbeständigen und filmbildenden Eigenschaften der gelierten Ölzusammensetzung zu einem außerordentlich übertragungsbeständigen und damit klimabeständigen Film führen, dessen Gesamtwirkung über die Einzelwirkung hinausgeht und daher eine synergistische Wirksamkeit zeigt.

Der wasserabsorbierende Puder ist vorteilhaft ein solcher auf Basis natürlicher pflanzlicher zellulosereicher Bestandteile, z.B. Bambuspuder, Baumwollpuder, Holzpuder, Guargummi, Xanthangummi etc. oder auf Basis anderer Substanzen wie Maltodextrin, Stärke, Stärkederivaten und Poly-acrylaten einschließlich solcher wie eines Stärke/Acrylamid/ Natriumacrylat-Copolymeren. Auch aus Acrylmonomeren hergestellte synthetische Polymere können dafür eingesetzt werden.

Besonders bevorzugt für den wasserabsorbierenden Puder sind 2 bis 8 Gew-%.

Als Verdickungsmittel kann bevorzugt ein auf einem Öl basierender Komplex verwendet werden, wie z.B. 12-Hydroxystearinsäure, hochmolekulare Kohlenwasserstoffe, Polyethylen, natürliche und synthetische Wachse, Säuren und Ester mit Schmelzpunkten im Bereich von 60 bis 100°C.

Als Lösungsmittel oder Trägerstoffe können eingesetzt werden polare und nichtpolare Öle, Kohlenwasserstoffe, Ether, Ester sowie Alkohole wie alkoxylierte Alkohole, mehrwertige Alkohole und Polyole. Zu Beispielen davon gehören Ethylalkohol, Isopropanol, Propylenglycol, Dipropylenglycol, Ethylenglycol, Glycerin, Diacetin, Triacetin, Isopropylpalmitat, Isododecan, Isohexadecan, Triglyceride und Mineralöl.

Zusätzlich kann der Komplex vernetzte Siliconpolymere enthalten, die eine bessere hydrophobe Abwaschbeständigkeit ergeben. Zu Beispielen dafür gehören Silicone, die ein Polymernetzwerk ergeben; vernetzte Dimethylpolysiloxan-Elastomere und deren Gemische in flüchtigem Siliconöl; Dimethyl/Vinyldimethicone Crosspolymer; Dimethicone/ Phenyl Vinyl Dimethicone Crosspolymer; vernetzte Silicon-Polyether-Copolymere mit Cyclopentasiloxan; Dimethicone Crosspolymer.

Der erfindungsgemäße Komplex kann als wesentlicher Bestandteil in Produkte der dekorativen Kosmetik eingearbeitet werden, wie z.B. Grundierungen, Lotionen, Lippenstifte, Lidschatten, Rouge, Make-up, Lippenglanz sowie auch in Cremes, Reinigungsflüssigkeiten, Körpershampoos, Sonnenschutzpräparate, Aftershaves und Deodorantpräparate, und verleiht diesen Produkten die gewünschte Klimabeständigkeit, verbessertes Transferverhalten, Beständigkeit auf der Haut und wasserabweisendes Verhalten.

Diese Produkte der dekorativen Kosmetik können weitere Wirk- und Hilfsstoffe enthalten. Beispielsweise können Mittel zur Verbesserung der Hautpenetrierung eingearbeitet werden, die eine verbesserte Ablagerung von Wirkstoffen ermöglichen. Zu diesen Verstärkern gehören Ethoxydiglycol, Panthenol und Phytantriol.

Der Anteil des klimabeständigen kosmetischen Komplexes in einem Produkt der dekorativen Kosmetik kann im Bereich von 0,1 bis 99,9 Gew.-% liegen. Bevorzugt ist der Einsatz im Bereich von 5 bis 40 Gew.-%, insbesondere 5-20 Gew-%.

Die Produkte der dekorativen Kosmetik können neben dem erfindungsgemäßen Komplex weitere Hilfs- und Wirkstoffe enthalten. Dazu gehören beispielsweise Pigmente, Farbstoffe, Antioxidationsmittel, Konservierungsmittel, weitere Feuchthaltemittel, Erweichungsmittel, Duftstoffe, Stabilisatoren, Adstringentien, Zellumwandlungsbeschleuniger, Zellwachstumsstimulatoren, antiinflammatorische Mittel, anti-mikrobielle Mittel, Hormonregulatoren, Enzyminhibitoren, UV-Absorber, Sonnenschutzmittel usw..

Weiterhin betrifft die Erfindung die Verwendung des erfindungsgemäßen kosmetischen Komplexes in kosmetischen Zusammensetzungen für den Schutz der Haut gegen witterungsbedingte negative Umwelteinflüsse.

Dabei liegt der Anteil des Komplexes in einem kosmetischen Produkt im Bereich von 5 bis 80 Gew-%, vorzugsweise 5-40 Gew-%, bezogen auf das Gesamtgewicht der kosmetischen Zusammensetzung.

Die Erfindung soll nachstehend durch Beispiele näher erläutert werden. Alle Angaben in % sind Gew.-%, sofern nichts anderes angegeben ist.

In der dazugehörigen Zeichnung zeigt

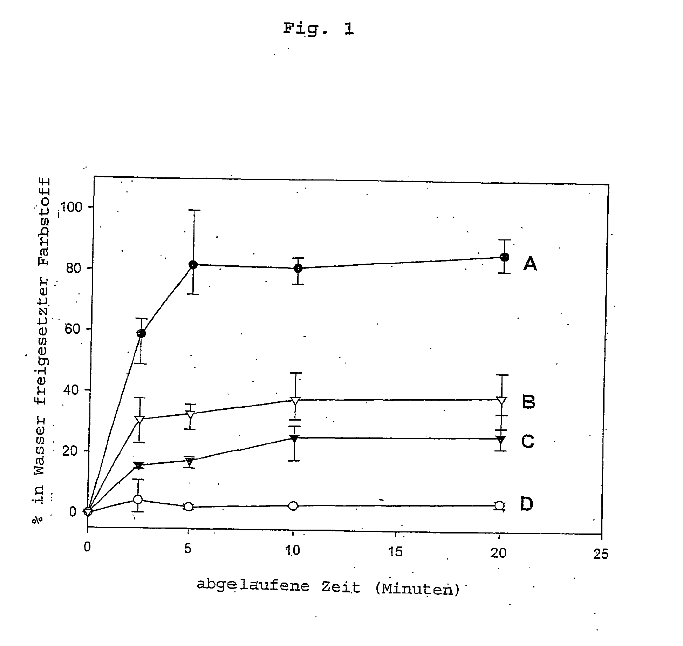

Fig. 1: ein Diagramm mit der prozentualen Farbstofffreisetzung in Wasser durch eine Fläche (Film) hindurch, die durch den erfindungsgemäßen Komplex geschützt ist.

Beispiel 1 **Klimabeständiger Komplex I**

Viskosität= 8000-10000 Pa.s (cps)

| Phase | Bestandteile | Gew-% |
|---|---|---|
| A | Isododecane, Ethylen/Propylen/Styren Copolymer, und Butylen/Ethylen/Styren Copolymer | 40-50 |
| | Isododecane | 20-30 |
| B | Trimethylpentanediol/Adipinsäure/Glycerin Copolymer | 5-15 |
| | Methylheptyl Isostearate | 5-15 |
| C | Di-C12-15 Alkyl Fumarate | 0,5-1,5 |
| | Behenoyl Stearic Acid | 0,5-1,5 |
| | Cyclomethicone und Dimethicone Copolymer | 5-15 |
| D | Bambuspuder | 1-5 |
| E | Konservierungsmittel | 0,3-1,0 |
| | Parfüm | 0,3-1,2 |
| | TOTAL | **100,0** |

Die Verfahrensweise für die Formulierung des Komplexes ist die folgende:

Die Bestandteile der Phase A werden in einen sauberen, trockenen, rostfreien Stahlbehälter gegeben, der mit einem Rührer ausgestattet ist. Unter langsamem Rühren wird das Gemisch langsam auf 80°C erhitzt und die Temperatur gehalten bis das Gemisch eine gleichmäßige Verteilung zeigt und keine ungelösten Rohmaterialien mehr vorhanden sind.

In einem separaten Behälter aus rostfreiem Stahl werden die Bestandteile der Phase B bis zur gleichmäßigen Verteilung vorgemischt. Die Phase B wird zu der Phase A mit moderater Rührgeschwindigkeit gegeben. Das Gemisch wird weiter gerührt und die Temperatur bei 75°C bis zur gleichmäßigen Verteilung gehalten. Unter fortwährendem Rühren wird die Phase C zu den Phasen A+B gegeben und die Mischungstemperatur weiterhin bei 75°C bis zur gleichmäßigen Verteilung gehalten.

Nach Unterbrechung der Erwärmung wird auf 50°C abgekühlt. Bei Erreichen der Temperatur von etwa 50 °C wird dem Gemisch die Phase D zugesetzt und alles zusammen bis zur Homogenität vermischt. Schließlich wird die Phase E zugesetzt und wiederum bis zur Homogenität gerührt.

Beispiel 2 **Klimabeständiger Komplex II**

Viskosität= 12000-15000 Pa.s (cps)

| Phase | Bestandteile | Gew-% |
|---|---|---|
| A | Hydrogenated Isobutene, Hydrogenated Styrene/Isoprene Copolymer, und Hydrogenated Styrene/Butadiene Copolymer | 55-65 |
| | Isododecane | 10-20 |
| B | Trimethylpentanediol/Adipinsäure/Glycerin Copolymer | 10-20 |
| | Hydrogenated Polydecene | 5-15 |
| C | Polyethylen | 0,5-1,5 |
| D | Modifizierte Stärke | 1-5 |
| E | Konservierungsmittel | 0,3-1,0 |
| | Parfüm | 0,3-1,2 |
| | **Gesamt** | **100,0** |

Die Herstellung erfolgt wie im Beispiel 1.

Beispiel 3 **Klimabeständiger Komplex III**

Viskosität= 22,000-30,000 Pa.s (cps)

| Phase | Bestandteile | Gew-% |
|---|---|---|
| A | Isononyl Isononanoate, Ethylen/Propylen/ Styren Copolymer, und Butylene/Ethylen/ Styren Copolymer | 20-30 |
| | Isopropyl Palmitate | 20-30 |
| B | Adipinsäure/Diethylenglycol/Glycerin Copolymer | 15-25 |
| | Hydrogenated Polydecene | 15-25 |
| C | Synthetischer Wachs | 2,5-10 |
| D | Bambuspuder | 1-5 |
| E | Konservierungsmittel | 0,3-1,0 |
| | Parfüm | 0,3-1,2 |
| | Gesamt | **100,0** |

Die Herstellung erfolgt wie im Beispiel 1.

Beispiel 4 **Vergleichsversuch 1**

Es wird eine Basislotion hergestellt. Zu der Basislotion werden unterschiedliche Anteile des klimabeständigen Komplexes I gegeben.

Formel der Basislotion

| Phase A | Wasser | 75-85 |
|---|---|---|
| | Glycerine USP Acrylates/C10-30 Alkyl Acrylate | 1-5 |
| | Crosspolymer | 0,1-0,5 |
| | Polysorbate 60 | 0,2-0,7 |
| Phase B | Mineralöl | 3-8 |
| | Stearylalkohol | 0,5-4 |
| | Cetylalkohol | 1-5 |
| | Glyceryl Monostearate | 1-5 |
| | Octyl Palmitate | 1-5 |
| Phase C | Wasser | 1-5 |
| | Triethanolamin 99% | 0.1-0.5 |
| Phase D | Phenonip | 0.8-1.2 |
| | Gesamt | **100.0** |

Die Verfahrensweise zur Formulierung der Basislotion ist folgende:

Die Bestandteile der Phase A werden in einen sauberen, trockenen, rostfreien Stahlbehälter gegeben, der mit einem Rührer ausgestattet ist. Unter langsamem Rühren wird mit der Erwärmung begonnen und das Gemisch auf 75°C erhitzt und bis zur gleichmäßigen Verteilung aller Bestandteile gehalten.

In einem separaten Behälter aus rostfreiem Stahl werden die Bestandteile der Phase B bis zur gleichmäßigen Verteilung vorgemischt. Die Phase B wird zu der Phase A mit moderater Rührgeschwindigkeit gegeben. Das Gemisch wird etwa 20 Minuten oder länger weiter gerührt und die Temperatur bei 75°C bis zur gleichmäßigen Verteilung gehalten.

Unter fortwährendem Rühren wird die Phase C zu den Phasen A+B gegeben und die Mischungstemperatur weiterhin bei 75°C bis zur gleichmäßigen Verteilung gehalten. Nach Unterbrechung der Erwärmung wird auf 50°C abgekühlt. Bei Erreichen der Temperatur von etwa 50 °C wird dem Gemisch die Phase D zugesetzt und alles zusammen bis zur Homogenität vermischt. Schließlich wird die Phase E zugesetzt und wiederum bis zur Homogenität gerührt.

Mit der Basislotion werden die Testlotionen A,B,C und D hergestellt; 0, 10, 20 und 70 Gew-% des klimabeständigen Komplexes werden jeweils hinzugegeben.

0,1 Gramm jeder Probe wird gleichmäßig auf eine Glasscheibe gesprüht, auf der 20 mg von 0,2%-igem Blue #1-Farbe aufgetragen ist. Die Scheibe wirde bei 50°C für 20 Minuten getrocknet und anschließend auf Umgebungstemperatur abgekühlt.

**Testverlauf:** Jede vorbereitete Scheibe wird in 25 g Wasser für eine gewünschte Zeit getaucht. Nach Entfernung der Scheibe wirde die verbliebene Wasserlösung mittels UV-VIS Spektrophotometer analysiert, um die Farbintensität zu ermitteln. **Testergebnisse:**

TABELLE 1

Klimabeständiger Filmschutz: Verhinderung von in Wasser freigesetztem Farbstoff (%)

| Min | kein Komplex (A) | 70% Komplex (D) | 20% Komplex (C) | 10% Komplex (B) |
|---|---|---|---|---|
| 0 | 0,06±0,13 | 0,0001±0,03 | 0,03±0,15 | 0,03±0,15 |
| 2,5 | 58,78±8,55 | 4,23±5,84 | 15,69±1,01 | 30,78±7,38 |
| 5 | 81,70±15,55 | 2,19±0,77 | 17,34±2,09 | 32,78±4,54 |
| 10 | 81,01±4,70 | 2,99±0,32 | 25,31±6,56 | 37,73±7,97 |
| 20 | 85,77±5,52 | 4,07±1,17 | 26,04±6,60 | 38,84±9,21 |

Diese Ergebnisse sind auch in Fig. 1 dargestellt. Die signifikante Absenkung der Prozente Farbstoff, die in Wasser freigesetzt wurden, um nahezu 50% bereits durch eine Lotion mit nur 10% klimabeständigem Komplex demonstriert die ausgezeichneten Wasserbeständigkeits- und Klimaschutz-Eigenschaften des erfindungsgemäßen Komplexes.

Beispiel 5 **Vergleichsversuch 2**

Es wird eine Basislotion gemäß Beispiel 4 hergestellt. Zu dieser Basislotion werden 10% des klimabeständigen Komplexes I, II bzw. III gegeben.

0,1 g der Probe wird gleichmäßig auf eine Glasscheibe gesprüht, die 20 mg 0,2% blauen Farbstoff Blue #1 trägt. Die Glasscheibe wird bei 50°C für 20 Minuten getrocknet und dann auf Raumtemperatur abgekühlt.

Der Testablauf entspricht dem in Beispiel 4. Der prozentuale Anteil des in dem Film zurückgebliebenen Farbstoffes wird wie folgt berechnet:

```
% Farbstoff, zurückgehalten im Film = 100%  minus (Farbintensität in der verbliebenen Wasserlösung / Farbintensität von  20 mg
von 0,2% Blue #1 in 25 g Wasser)
```

**Testergebnisse:**

Tabelle 2

Klimabeständiger Filmschutz: zurückgehaltener Farbstoff (%) im Schutzfilm

| 10% Komplex I | 10% Komplex II | 10% Komplex III | ohne Komplex |
| A | B | C | D |
|---|---|---|---|
| 74,0 ± 6,6 | 39,2 ± 9,5 | 38,9 ± 4,2 | 14,2 ± 5,5 |

Diese Ergebnisse zeigen, daß mit 10% Komplex bereits der 2,5- bis 5-fache Schutz im Vergleich mit einer Basislotion ohne Zusatz eines solchen Komplexes erreicht wird.

Beispiel 6 **Klimabeständiger Komplex IV**

Viskosität = 85000-150000 Pa.s (cps)

| Phase | Bestandteile | Gew-% |
|---|---|---|
| A | Hydrogenated Isobutene, Hydrogenated Styrene/Isoprene Copolymer und Hydrogenated Styrene/Butadiene Copolymer | 60-70 (65) |
| | Isododecane | 3-12 (4) |
| | Hydrogenated Polydecene | 5-10 (7,5) |
| B | Trimethylpentanediol/Adipic acid/Glycerine Copolymer | 15-25 (20) |
| C | Polyethylen | 0,5-1,5 (0,5) |
| D | Maltodextrin | 1-5 (2) |
| E | Konservierungsmittel | 0,5-1 (0,8) |
| | Parfüm | 0,5-1 (0,2) |
| | **Gesamt** | 100,0 |

Die Verfahrensweise zur Herstellung des Komplexes IV entspricht der von Beispiel 4. Die Zahlen in Klammern geben eine spezifische Zusammensetzung an, auch in den folgenden Beispielen.

Beispiel 7 **Hautschutzcreme**

Es wird eine Hautschutzcreme mit folgender Grundzusammensetzung hergestellt.

| Phase | Bestandteile | Gew-% |
|---|---|---|
| A | Wasser | 75-85 |
| | Propylenglycol | 2-8 |
| | Glycereth-7 Triacetate | 1-5 |
| | PEG/PPG-4/12 Dimethicone | 0,3-1 |
| B | Polyacrylamid | 0,3-1,5 |
| | C13-14 Isoparaffin | 0,1-1 |
| | Laureth-7 | 0,1-0,5 |
| | Dimethicone | 5-10 |
| | Isohexadecane | 1-5 |
| C | Wasser | 1-5 |
| | Triethanolamin 99% | 0,1-0,5 |
| D | Konservierungsmittel | 0,3-1 |
| | Parfüm | 0,3-1 |
| | UV Filter | 3-8 |
| | Gesamt | 100,0 |

Die Verfahrensweise zur Formulierung der Creme-Grundzusammensetzung ist folgende:

Die Bestandteile der Phase A werden in einen sauberen, trockenen, rostfreien Stahlbehälter gegeben, der mit einem Rührer ausgestattet ist. Unter langsamem Rühren wird mit der Erwärmung begonnen und das Gemisch auf 75°C erhitzt und bis zur gleichmäßigen Verteilung aller Bestandteile gehalten.

In einem separaten Behälter aus rostfreiem Stahl werden die Bestandteile der Phase B bis zur gleichmäßigen Verteilung vorgemischt. Die Phase B wird zu der Phase A mit moderater bis hoher Rührgeschwindigkeit gegeben. Das Gemisch wird etwa 20 Minuten oder länger weiter gerührt und die Temperatur bei 75°C bis zur gleichmäßigen Verteilung gehalten.

Unter fortwährendem Rühren wird die Phase C zu den Phasen A+B gegeben und die Mischungstemperatur weiterhin bei 75°C bis zur gleichmäßigen Verteilung gehalten. Nach Unterbrechung der Erwärmung wird auf 50°C abgekühlt. Bei Erreichen der Temperatur von etwa 50 °C wird dem Gemisch die Phase D zugesetzt und alles zusammen bis zur Homogenität vermischt.

Zu diese Grundzusammensetzung werden 30% des klimabeständigen Komplexes IV gegeben. Anschließend erfolgt ein Test entsprechend dem Vergleichsversuch 2.

**Testergebnisse:**

**Tabelle 3**

Klimabeständiger Filmschutz: zurückgehaltener Farbstoff (%) im Schutzfilm

| 30% Komplex IV | ohne Komplex |
|---|---|
| **A** | **B** |
| $57,6 \pm 7,3$ | $0,3 \pm 2,2$ |

Die Ergebnisse zeigen, daß mit dem Komplex etwa das 5-fache des Schutzes erreicht wird im Vergleich ohne Zusatz eines solchen Komplexes.

Beispiel 8 **Klimabeständiger Komplex V**

Viskosität = 400,000-800,000 Pa.s (cps)

| Phase | Bestandteile | Gew-% |
|---|---|---|
| A | Isononyl Isononanoate und Ethylen/Propylen/ Styren Copolymer, und Butylene/Ethylen/ Styren Copolymer | 35-50 (40) |
| | Hydrogenated Polydecene | 20-35 (28) |
| B | Adipinsäure/Diethylenglycol/Glycerin Copolymer | 15-25 (20) |
| | Isopropyl Palmitate | 2-8(5) |
| C | Synthetischer Wachs | 1-5 (3) |
| D | Xanthan Gum und Guar Gum | 1-5(3) |
| E | Konservierungsmittel | 0,5-1(0,8 |
| | Parfüm | 0,5-1(0,2 |
| | **Gesamt** | 100,0 |

Die Verfahrensweise zur Formulierung des Komplexes entspricht der von Beispiel 6.

Beispiel 9 **Sensitives Körperwaschmittel**

Zur Herstellung eines Körperwaschmittels werden folgende Grundbestandteile vermischt:

| Phase | Bestandteile | Gew-% |
|---|---|---|
| A | Wasser | 65-80 |
| | Glycerin | 1-5 |
| | Propylenglycol | 0,5-3 |
| | Sodium Laureth Sulfate | 10-20 |

(fortgesetzt)

| Phase | Bestandteile | Gew-% |
|---|---|---|
| | Sodium Lauryl Sulfate | 1-3 |
| | Coco-Betaine | 1-5 |
| | PPG-1-PEG-9 Lauryl Glycol Ether | 0,5-5 |
| | PEG-7 Glyceryl Cocoate | 0,5-5 |
| | PEG-55 Propylene Glycol Oleate | 0,5-5 |
| B | Natriumchlorid | 0,5-5 |
| | Citronensäure (zur Einstellung auf pH 5,5-6,0) | q.s. |
| C | Konservierungsmittel | 0,3-1 |
| | Parfüm | 0,3-1 |
| | UV-Filter | 4-12 |
| | Gesamt | 100,0 |

Die Bestandteile der Phase A werden in einen sauberen, trockenen, rostfreien Stahlbehälter gegeben, der mit einem Rührer ausgestattet ist. Unter langsamem Rühren wird das Gemisch langsam auf 80°C erhitzt und die Temperatur gehalten bis das Gemisch eine gleichmäßige Verteilung zeigt und keine ungelösten Rohmaterialien mehr vorhanden sind.

In einem separaten Behälter aus rostfreiem Stahl werden die Bestandteile der Phase B unter Erwärmung auf 65-70 °C und weiterer Erwärmung bis zur gleichmäßigen Verteilung vorgemischt. Die Phase B wird zu der Phase A mit moderater Rührgeschwindigkeit gegeben. Das Gemisch wird zwecks gleichmäßiger Erteilung weiter 20 Minuten gerührt. Dann wird das Gemisch auf 50 °C abgekühlt, und unter fortwährendem Rühren wird die Phase C zu den Phasen A+B gegeben und die Mischung weiter bis zur gleichmäßigen Verteilung und Homogenität gerührt.

Zu diesem Basisgemisch eines Körperwaschmittels werden 30% des klimabeständigen Komplexes V gegeben. Anschließend erfolgte ein Test entsprechend dem Vergleichsversuch 2.

**Testergebnisse:**

**Tabelle 4**

Klimabeständiger Filmschutz:
zurückgehaltener Farbstoff (%) im Schutzfilm

| 30% Komplex V | ohne Komplex |
|---|---|
| A | B |
| 33,6 $\pm$ 12,7 | 8,1 $\pm$ 2,7 |

Mit dem Komplex wird ein etwa 4-facher Schutz erreicht im Vergleich zu der kosmetischen Zusammensetzung ohne den Komplex.

Beispiel 10 **Komplex VI**

Viskosität = 50,000-90,000 Pa.s (cps)

| Phase | Bestandteile | Gew-% |
|---|---|---|
| A | Isopropyl Palmitate, Ethylen/Propylen/Styren Copolymer und Butylene/Ethylen/Styren Copolymer | 40-50 (45) |
| | Isododecan | 25-35 (30) |
| B | Trimethylpentanediol/Adipinsäure/Glycerin Copolymer | 15-25 (20) |
| C | Synthetischer Wachs | 2,5-8(2) |
| D | Natürlicher Baumwollpuder | 1-5(2) |
| E | Konservierungsmittel | 0,3-1(0,8) |

(fortgesetzt)

| Phase | Bestandteile | Gew-% |
|---|---|---|
| | Parfüm | 0,3-1(0,2) |
| **Total** | | 100,0 |

Die Verfahrensweise zur Formulierung des Komplexes entsprach der von Beispiel 6.

Beispiel 11 **Sonnenschutzzubereitung mit SPF 20**

| Phase | Bestandteile | Gew-% |
|---|---|---|
| A | Wasser | 55-65 |
| | Propylenglycol | 2-8 |
| | Sodium polyacrylate | 1-5 |
| | Tween 60 | 1-5 |
| B | Ethylhexyl Methoxy Cinnamate | 6-7,5 |
| | Benzophenone-3 | 3,5-4,5 |
| | Ethylhexyl Salicylate | 4,5-5,5 |
| | Octocrylene | 6-7,5 |
| | PPG-15 Stearyl Ether | 1-5 |
| | Alkyl Siloxane Wax | 1-5 |
| | Hexyl Laurate | 0,5-3 |
| | Polyglyceryl-4 Isostearate | 0,5-3 |
| | Cetyl PEG/PPG-70/1 Dimethicone | 0,5-3 |
| | Trilaureth-4 Phosphate | 1-5 |
| | Dimethicone | 1-10 |
| C | Konservierungsmittel | 0,3-1 |
| | Parfüm | 0,3-1 |
| | UV Absorptionsmittel | q.s. |
| | Total | 100,0 |

Die Verfahrensweise zur Herstellung der Sonnenschutzzubereitung ist wie folgt.

Die Bestandteile der Phase A werden in einen sauberen, trockenen, rostfreien Stahlbehälter gegeben, der mit einem Rührer ausgestattet ist. Unter langsamem Rühren wird mit der Erwärmung begonnen und das Gemisch auf 65-70 °C erhitzt und bis zur gleichmäßigen Verteilung aller Bestandteile gehalten.

In einem separaten Behälter aus rostfreiem Stahl werden die Bestandteile der Phase B bis zur gleichmäßigen Verteilung bei etwa 65 °C vorgemischt. Die Phase B wurde zu der Phase A mit moderater bis hoher Rührgeschwindigkeit gegeben. Das Gemisch wird etwa 20 Minuten oder länger weiter gerührt und die Temperatur bis zur gleichmäßigen Verteilung gehalten.

Unter fortwährendem Rühren wird die Phase C zu den Phasen A+B gegeben und die Mischungstemperatur weiterhin bei 65°C bis zur gleichmäßigen Verteilung gehalten. Nach Unterbrechung der Erwärmung wird auf 50°C abgekühlt. Bei Erreichen der Temperatur von etwa 50 °C wird dem Gemisch die Phase D zugesetzt und alles zusammen bis zur Homogenität vermischt.

Zu diesem Basisgemisch eines Sonnenschutzmittels werden 30% des klimabeständigen Komplexes VI gegeben. Anschließend erfolgt ein Test entsprechend dem Vergleichsversuch 2.

**Testergebnisse:**

**Tabelle 5**

Klimabeständiger Filmschutz:
zurückgehaltener Farbstoff (%) im Schutzfilm

| 30% Komplex VI | ohne Komplex |
|---|---|
| **A** | **B** |
| 49,3 ± 13,5 | 19,4 ± 11,3 |

Mit dem Komplex wird etwa der 2,5-fache Schutz erreicht im Vergleich zu dem Mittel ohne den Komplex.

Insgesamt zeigen die Beispiele und die durchgeführten Tests eine signifikante Verbesserung der Wasserbeständigkeit. Die Tests zur Feuchthaltewirkung zeigten ähnlich gute Ergebnisse, insbesondere sehr langanhaltende Wirkungen im Zeitraum bis 24 Stunden.

Bevorzugte Viskositäten für den erfindungsgemäßen klimabeständigen Komplex liegen zwischen 50000 und 500000 Pa.s, gemessen nach Brookfield mit Spindel TC/TD/TE bei 25°C und im Bereich von 50-75 % der Spindelgeschwindigkeiten.

**Patentansprüche**

1. Klimabeständiger kosmetischer Komplex, **dadurch gekennzeichnet, dass** er
0,1 - 90 Gew.-% einer gelierten Ölzusammensetzung, bestehend aus einer Ölkomponente und einer Polymerkomponente, wobei die Polymerkomponente ausgewählt ist aus der Gruppe, bestehend aus Tri-Block-Copolymeren, Sternpolymeren, Radialpolymeren und Multi-Block-Polymeren von Polystyrol, Polyethylen, Polyvinylchlorid, Polyisopren, Polybutadien, Ethylen/Butadien-Copolymeren, Ethylen/Propylen-Copolymeren, Ethylen/Butylen-Copolymeren, Ethylen-Propylen/Dien-Copolymeren, Styrol-Ethylen/Propylen-Copolymeren, Styrol-Ethylen/Butadien-Copolymeren, Styrol-Isopren-Copolymeren, Styrol-Butadien-Copolymeren, Styrol-Ethylen/Propylen-Styren-Copolymeren, Styrol-Ethylen/Butadien-Styren-Copolymeren, Styrol-Isopren-Styrol-Copolymeren, Styrol-Butadien-Styrol-Copolymeren und Gemischen davon;
0,1 - 80 Gew.-% eines topischen wasserabweisenden vernetzten Polyesters mit einem Molekulargewicht von 600 - 8000, bestehend aus mehrwertigen Alkoholen und Dicarbonsäuren; 0,01 - 20 Gew.-% eines wasserabsorbierenden Puders mit einer Teilchengröße von 1 bis 100 $\mu$m, wobei der Puder aus der Gruppe ausgewählt ist, bestehend aus Pudern auf Basis natürlicher pflanzlicher zellulosereicher Bestandteile, Maltodextrin, Stärke, Stärke/Polyacrylat-Copolymere, aus Acrylmonomeren hergestellte synthetische Polymere und Gemische davon;
0,01 - 20 Gew.-% eines Verdickungsmittels;
0,1 - 50 Gew.-% organische Lösungsmittel, Trägerstoffe oder Gemische davon
und keinen Emulgator enthält.

2. Klimabeständiger Komplex nach Anspruch 1, **dadurch gekennzeichnet, dass** der Bereich für die gelierte Ölzusammensetzung 20 bis 70 Gew-% beträgt.

3. Klimabeständiger Komplex nach Anspruch 1, **dadurch gekennzeichnet, dass** der Polymeranteil in der Ölzusammensetzung im Bereich von 1:5 bis 1:50 Polymerkomponente:Ölkomponente liegt.

4. Klimabeständiger Komplex nach Anspruch 1, **dadurch gekennzeichnet, dass** die Ölkomponente ausgewählt ist aus der Gruppe, bestehend aus Kohlenwasserstoffen, Fettalkoholen, natürlichen und synthetischen Ölen, Estern, Ethern und Gemischen davon.

5. Klimabeständiger Komplex nach Anspruch 1, **dadurch gekennzeichnet, dass** das Polymere ausgewählt ist aus der Gruppe, bestehend aus Diblockcopolymeren, Triblockcopolymeren und Gemischen davon.

6. Klimabeständiger Komplex nach Anspruch 1, **dadurch gekennzeichnet, dass** der Bereich für die wasserabweisende Substanz 5 bis 30 Gew-% beträgt.

7. Klimabeständiger Komplex nach Anspruch 1, **dadurch gekennzeichnet, dass** der Komplex 0,01 bis 50 Gew-% vernetzte Siliconpolymere enthält.

8. Kosmetische Zusammensetzung, enthaltend 5 bis 80 Gew-%, bevorzugt 10-40 Gew-%, bezogen auf das Gesamtgewicht der kosmetischen Zusammensetzung eines klimabeständigen Komplexes gemäß Anspruch 1.

9. Verwendung eines klimabeständigen Komplexes nach den Ansprüchen 1-7 in kosmetischen Zusammensetzungen für den Schutz der Haut gegen witterungsbedingte negative Umwelteinflüsse.

10. Verwendung nach Anspruch 9, **dadurch gekennzeichnet, dass** der Komplex in einem Produkt der dekorativen Kosmetik vorliegt, ausgewählt unter Grundierungen, Lotionen, Lippenstiften, Lidschatten, Rouge, Make-up, Lippenglanz.

11. Verwendung nach Anspruch 9, **dadurch gekennzeichnet, dass** der Anteil des Komplexes in einer kosmetischen Zusammensetzung im Bereich von 5 bis 80 Gew-% liegt, vorzugsweise 5-40 Gew-%, bezogen auf das Gesamtgewicht der kosmetischen Zusammensetzung.

## Claims

1. Climaproof cosmetic complex, **characterized in that** it contains
0.1 to 90 % by weight of a gelled oil composition consisting of an oil component and a polymer component, which polymer component is selected from the group consisting of tri-block copolymers, star polymers, radial polymers and multi-block polymers of polystyrene, polyethylene, polyvinyl chloride, polyisoprene, polybutadiene, ethylene/butadiene copolymers, ethylene/propylene copolymers, ethylene/butylenes copolymers, ethylene-propylene/diene copolymers, styrene-ethylene/propylene copolymers, styrene-ethylene/butadiene copolymers, styrene-isoprene copolymers, styrene-butadiene copolymers, styrene-ethylene/propylene-styrene copolymers, styrene-ethylene/butadiene-styrene copolymers, styrene-isoprene-styrene copolymers, styrene-butadiene-styrene copolymers, and mixtures thereof;
0.1 to 80 % by weight of a topic water-repellent cross-linked polyester having a molecular weight of 600 to 8000 and consisting of polyvalent alcohols and mono- or dicarbon acids;
0.01 to 20 % by weight of a water-absorbing powder having a particle size of 1 to 100 $\mu$m, which powder is selected from the group consisting of powders based on natural plant constituents rich in cellulose, maltodextrine, starch, starch/polyacrylate copolymers, synthetic polymers made from acrylic monomers and mixtures thereof;
0.01 to 20 % by weight of a thickening agent; and
0.1 to 50 % by weight of organic solvents, carrier substances, or mixtures thereof,
and that it does not contain an emulsifier.

2. Climaproof complex according to claim 1, **characterized in that** the range for the gelled oil composition is 20 to 70 % by weight.

3. Climaproof complex according to claim 1, **characterized in that** the polymer share in the oil composition is in the range of 1:5 to 1:50 polymer component:oil component.

4. Climaproof complex according to claim 1, **characterized in that** the oil component is selected from the group sonsiting of hydrocarbons, fatty alcohols, natural and synthetic oils, esters, ethers, and mixtures thereof.

5. Climaproof complex according to claim 1, **characterized in that** the polymer is selected from the group consisting of diblock copolymers, tri-block copolymers, and mixtures thereof.

6. Climaproof complex according to claim 1, **characterized in that** the range for the water-repellent substance is 5 to 30 % by weight.

7. Climaproof complex according to claim 1, **characterized in that** the complex contains 0.01 to 50 % by weight cross-linked silicone polymers.

8. Cosmetic composition containing 5 to 80 % by weight, preferred 10 to 40 % by weight, related to the total weight of the cosmetic composition, of a climaproof complex according to claim 1.

9. Use of a climaproof complex according to claims 1 to 7 in cosmetic compositions for skin protection against weather-related negative environmental influences.

**10.** Use according to claim 9, **characterized in that** the complex is present in a product of decorative cosmetic selected from the group consisting of foundations, lotions, lipsticks, eye shadows, rouges, make-up's and lip glosses.

**11.** Use according to claim 9, **characterized in that** the percentage of the complex in a cosmetic composition is in the range of 5 to 80 % by weight, preferred 5 to 40 % by weight, related to the total weight of the cosmetic composition.


**Revendications**

**1.** Complexe cosmétique résistant aux conditions climatiques, **caractérisé en ce qu'**il contient
0,1 à 90 % en poids d'une composition d'huile fournie, constituée d'un composant huile et d'un composant polymère, moyennant quoi le composant polymère est choisi parmi le groupe constitué des copolymères triblocs, des polymères en étoile, des polymères radiaux et des polymères multiblocs de polystyrène, polyéthylène, chlorure de polyvinyle, polyisoprène, polybutadiène, copolymères éthylène/butadiène, copolymères éthylène/propylène, copolymères éthylène/butylène, copolymères éthylène-propylène/diène, copolymères styrène-éthylène/propylène, copolymères styrène-éthylène/butadiène, copolymères styrène-isoprène, copolymères styrène-butadiène, copolymères styrène-éthylène/propylène-styrène, copolymères styrène-éthylène/butadiène-styrène, copolymères styrène-isoprène-styrène, copolymères styrène-butadiène-styrène et de mélanges d'entre eux,
0,1 à 80 % en poids d'un polyester réticulé hydrophobe topique ayant un poids moléculaire de 600 à 8000, constitué d'alcools plurivalents et d'acides dicarboxyliques,
0,01 à 20 % en poids d'une poudre absorbant l'eau, ayant une granulométrie de 1 à 100 $\mu$m, moyennant quoi la poudre est choisie dans le groupe constitué de poudres à base de constituants végétaux naturels riches en cellulose, de maltodextrine, d'amidon, de copolymères amidon-polyacrylate, de polymères synthétiques fabriqués à partir de monomères d'acryle et de mélanges d'entre eux,
0,01 à 20 % en poids d'un agent épaississant,
0,1 à 50 % en poids d'un solvant organique, de substances de support ou de mélanges d'entre eux, et
qu'il ne contient aucun émulsifiant.

**2.** Complexe résistant aux conditions climatiques selon la revendication 1,
**caractérisé en ce que** la plage pour la composition huile fournie est de 20 à 70 % en poids.

**3.** Complexe résistant aux conditions climatiques selon la revendication 1,
**caractérisé en ce que** la proportion de polymère dans la composition huile est dans une gamme de 1 : 5 à 1 : 50, en considérant le rapport composant polymère : composant huile.

**4.** Complexe résistant aux conditions climatiques selon la revendication 1,
**caractérisé en ce que** le composant huile est choisi parmi le groupe comprenant les hydrocarbures, les alcools gras, les huiles naturelles et synthétiques, les esters, les éthers et les mélanges d'entre eux.

**5.** Complexe résistant aux conditions climatiques selon la revendication 1,
**caractérisé en ce que** le polymère est sélectionné parmi le groupe constitué des copolymères diblocs, des copolymères triblocs et des mélanges d'entre eux.

**6.** Complexe résistant aux conditions climatiques selon la revendication 1,
**caractérisé en ce que** la proportion de la substance hydrophobe est dans une gamme de 5 à 30 % en poids.

**7.** Complexe résistant aux conditions climatiques selon la revendication 1,
**caractérisé en ce que** le complexe contient 0,01 à 50 % en poids de polymères de silicone réticulés.

**8.** Composition cosmétique, contenant 5 à 80 % en poids, de préférence de 10 à 40 % en poids rapporté au poids total de la composition cosmétique d'un complexe résistant aux conditions climatiques selon la revendication 1.

**9.** Utilisation d'un complexe résistant aux conditions climatiques selon les revendications 1 à 7 dans des compositions cosmétiques destinées à la protection de la peau contre des influences nocives de l'environnement dues aux conditions météorologiques.

**10.** Utilisation selon la revendication 9, **caractérisée en ce que** le complexe est présent dans un produit cosmétique à effet esthétique, choisi parmi les fonds de teint, les lotions, les rouges à lèvres, les fards à paupières, les rouges à joue, les maquillages, les rouges à lèvres ultra-brillants.

**11.** Utilisation selon la revendication 9, **caractérisée en ce que** la proportion du complexe dans une composition cosmétique est dans une plage de 5 à 80 % en poids, de préférence de 5 à 40 % en poids, rapporté au poids total de la composition cosmétique.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1013256 A
- WO 2004066918 A
- US 20030165451 A1
- EP 925778 B1
- EP 1064930 B1